# EUROPÄISCHE PATENTSCHRIFT

(11) **EP 3 048 996 B1**
(45) Veröffentlichungstag und Bekanntmachung des Hinweises auf die Patenterteilung: **19.09.2018**
(21) Anmeldenummer: 14783562.3
(22) Anmeldetag: 24.09.2014
(51) Int. Cl.: A61B 17/12, A61B 17/42

(54) **VORRICHTUNG ZUR VERWENDUNG ZUR THERAPIE EINER UTERINEN BLUTUNG**
DEVICE FOR USE FOR THE THERAPY OF UTERINE BLEEDING
DISPOSITIF DESTINÉ À ÊTRE UTILISÉ POUR TRAITER DES SAIGNEMENTS UTÉRINS

(30) Priorität: 24.09.2013 DE 102013219202
(43) Veröffentlichungstag der Anmeldung: 03.08.2016
(73) Patentinhaber: Meyer, Georg-Albrecht, 90471 Nürnberg (DE)
(72) Erfinder: Meyer, Georg-Albrecht, 90471 Nürnberg (DE)
(74) Vertreter: Dr. Gassner & Partner mbB
(86) Internationale Anmeldenummer: PCT/EP2014/070360
(87) Internationale Veröffentlichungsnummer: WO 2015/044197

(56) Entgegenhaltungen:
- WO-A1-90/12543
- FR-A1- 2 931 353
- GB-A- 2 441 112
- US-A1- 2012 172 898

## Beschreibung

Die Erfindung betrifft eine Vorrichtung zur Verwendung zur Therapie einer uterinen Blutung bei einem Menschen und ein Set, umfassend eine Mehrzahl solcher Vorrichtungen, zur Verwendung zur Therapie einer uterinen Blutung bei einem Menschen.

Bei einer normalen Geburt kontrahiert sich der Uterus (Gebärmutter) nach dem Ablösen der Plazenta und bewirkt durch die Kontraktion ein Stoppen der Blutung im Bereich der Plazentaablösung. Es besteht der Eindruck, dass mit zunehmender Medikation mit Tokolytika, Oxytocin sowie Prostaglandinen und mit zunehmender Häufigkeit von Entbindungen per Kaiserschnitt die Häufigkeit schwer zu stoppender Blutungen der Gebärmutter nach der Geburt (postpartale uterine Blutungen) zunimmt. Ursachen dafür können Implantationsstörungen, d. h. ein Einwachsen der Plazenta in die Gebärmutter, oder Plazentalösungsstörungen sein. Als Folge von nicht gestoppten postpartalen uterinen Blutungen kommt es noch immer zu Todesfällen. Schwer zu stoppende uterine Blutungen können auch im Zusammenhang mit anderen Ursachen oder Erkrankungen auftreten. Es besteht daher ein dringender Bedarf an geeigneten Maßnahmen zum Stoppen solcher Blutungen.

Nach einer Geburt ist eine erste übliche Maßnahme die Verabreichung von Medikamenten, welche eine Kontraktion der Gebärmutter bewirken. Dies kann jedoch ineffektiv sein, weil das Muskelgewebe der Gebärmutter, insbesondere nach lange dauernden Geburten, oft keine zur Stoppung der Blutung ausreichende Kontraktionsfähigkeit mehr aufweist.

Wenn sich diese Maßnahme als ineffektiv erweist, wird als nächstes üblicherweise ein manuelles Lösen von noch in der Gebärmutter verbliebener Plazenta oder Plazentateilen versucht. Kann auch dadurch ein Stoppen der Blutung nicht erreicht werden, wird üblicherweise eine Kürettage, d. h. eine Ausschabung der Gebärmutterhöhle vorgenommen. Wenn auch dadurch die postpartale Blutung nicht zu stoppen ist, kann eine sogenannte Ballontamponade angelegt werden. Dabei wird ein Katheter transvaginal in den Uterus eingeführt. Am Ende des Katheters ist ein mit einer sterilen Flüssigkeit zu füllender Ballon aus Silikon vorgesehen. Durch das Füllen des Ballons wird die Innenwand des Uterus komprimiert und dadurch eine Blutung möglicherweise gestoppt. Nachteilig ist dabei jedoch, dass der Uterus, der sich physiologisch nach einer Geburt eigentlich zusammenziehen soll, aufgedehnt wird. Dadurch ist es möglich, dass auch die Blutung verursachende eröffnete Gefäße weiter aufgedehnt werden und dadurch ein Stoppen der Blutung gerade verhindert wird. Weiterhin kann es schwierig sein, den Ballon bei eröffnetem Muttermund zu fixieren. Ein weiterer Nachteil dieser Maßnahme besteht darin, dass nach einem Kaiserschnitt gesetzte Nähte an der Gebärmutter durch Dehnung belastet werden und unter der Belastung sogar reißen können.

Wenn auch diese Maßnahme nicht zu einem Stoppen der Blutung führt, besteht eine weitere Maßnahme im Legen von sogenannten Uteruskompressionsnähten, wie beispielsweise B-Lynch-Nähten. Dafür müssen dem Uterus jedoch mehrere Stichverletzungen, die insbesondere bei Gerinnungsstörungen Anlass für weitere Blutungen sein können, zugefügt werden. Des Weiteren muss dazu der Bauchraum eröffnet werden, wenn er nicht bereits wegen eines Kaiserschnitts eröffnet ist.

Wenn auch dadurch die Blutung nicht zu stoppen ist, ist bisher die ultimative Maßnahme eine Notfall-Hysterektomie, d. h. die Entfernung der Gebärmutter. Diese Maßnahme kann zwar das Leben der Mutter retten, ist jedoch ein schwerer operativer Eingriff und weist den Nachteil des Verlusts der Gebärfähigkeit auf.

Aus der WO 2009/103298 A2 ist eine Vorrichtung aus zwei intraabdominal anzuwendenden Ballonen bekannt. Ein anteriorer Ballon davon soll an der anterioren Gebärmutterwand anliegen und ein posteriorer Ballon soll an der posterioren Gebärmutterwand anliegen. Auf der Oberseite können die beiden Ballone durch eine transparente dünne elastische Plastikfolie, die den Fundus des Uterus abdeckt, miteinander verbunden sein. Der Zweck der Vorrichtung ist es, die anteriore Uteruswand gegen die posteriore Uteruswand zu pressen, um dadurch eine uterine Blutung zu minimieren. Die Ballone können dazu mit Gas oder einem Fluid gefüllt werden.

Aus der FR 2 931 353 A1 ist ein Beutel für eine mechanische Kompression des Uterus nach einer Geburt bekannt. Der Beutel besteht aus einem resorbierbaren Geflecht mit einer Öffnung, durch die sich der Uterus einführen lässt. Der Beutel ist mit drei Schnüren ausgestattet, die durch ihr Festziehen die Ausübung einer Kompression auf die Gebärmutter im Beutel erlauben. Die Dauer der Ausübung der Kompression auf die Gebärmutter lässt sich dabei nicht individuell nach Bedarf steuern. Die Kompression endet, wenn der Beutel resorbiert ist oder sich die Gebärmutter durch Kompression oder Rückbildung verkleinert. Eine vorherige Beendigung der Kompression ist nicht möglich.

Aus der WO 90/12543 sind ein Verfahren und eine Vorrichtung zur Blutstillung und zum Einhüllen eines blutenden inneren Organs bekannt. Die Vorrichtung offenbart die in der Präambel des Anspruchs 1 definierten Merkmale und umfasst eine flexible, ein Kompartment definierende Struktur mit ausreichenden Ausmaßen, um zur Gesamtgeometrie eines wesentlichen Anteils des Organs zu passen. Diese Struktur hat eine innere und eine äußere Oberfläche und kann so konfiguriert werden, dass sie den wesentlichen Anteil des Organs umgibt und enthält. Die Vorrichtung kann mit einer äußeren Schicht und einer inneren Schicht sowie einer zwischen der äußeren Schicht und der inneren Schicht gebildeten Kammer ausgestattet sein. Die Kammer kann mit unter Druck stehendem Gas oder einer Flüssigkeit gefüllt werden, wodurch sich die innere Schicht ausdehnt und einen Druck auf das innere Organ ausübt. Bei dem inneren Organ kann es sich um eine Gebärmutter handeln. Wenn die Vorrichtung nicht mehr erforderlich ist, kann sie durch eine Operation wieder entfernt werden.

Insgesamt gibt es im Stand der Technik bisher keine befriedigende und gleichzeitig nebenwirkungsfreie oder -arme Maßnahme zur schnellen Stoppung einer starken uterinen Blutung, die nach dem Stoppen der Blutung ohne großen Aufwand wieder entfernt werden kann.

Aufgabe der vorliegenden Erfindung ist es daher, eine dazu geeignete Vorrichtung und ein dazu geeignetes Set anzugeben.

Die Aufgabe wird durch die Merkmale der Patentansprüche 1 und 14 gelöst. Zweckmäßige Ausgestaltungen ergeben sich aus den Merkmalen der Patentansprüche 2 bis 13.

Erfindungsgemäß ist eine Vorrichtung zur Verwendung zur Therapie einer, insbesondere postpartalen, uterinen Blutung bei einem Menschen vorgesehen. Die Vorrichtung umfasst eine Haube aus einem elastischen, sterilisierbaren Material, wobei die Haube eine Form aufweist, die es, insbesondere nach einer Geburt, erlaubt, die Haube von außen so über den Uterus zu stülpen, dass dieser dadurch komprimiert wird. Dazu ist es zwar erforderlich, den Unterbauch mittels Laparotomie (Bauchschnitt, insbesondere Unterbauchschnitt nach Pfannenstiel) zu eröffnen, die Gebärmutter bleibt durch diese Maßnahme jedoch unverletzt. "Von außen" bedeutet in diesem Zusammenhang also von der Außenseite des Uterus her, jedoch bei eröffnetem Bauchraum. Die Haube ist dabei so bemessen, dass eine ausreichende Kompression zur Stoppung oder zumindest starken Verminderung der Blutung auf den Uterus ausgeübt wird. Das Ausüben der Kompression kann von Anfang an durch die Haube erfolgen. Es kann aber auch dadurch erfolgen, dass der Uterus zunächst manuell komprimiert wird und die Haube dann über den Uterus gestülpt wird. Dabei übt die Haube dadurch eine Kompression auf den Uterus aus, dass der Uterus nach dem Überstülpen der Haube versucht, sich wieder auszudehnen und dabei gegen die Haube drückt, diese Ausdehnung jedoch durch die Haube verhindert oder zumindest begrenzt wird. Die Haube sollte dazu relativ fest sein, d. h. das Material und die Stärke des Materials, aus dem die Haube besteht, sollte so gewählt werden, dass keine oder keine wesentliche Ausdehnung des Uterus mehr möglich ist. Durch die Kompression kann sich die Gebärmutter nach der Geburt auch erholen, bis sie sich wieder von selbst, gegebenenfalls nach Gabe eines kontraktionsfördernden Medikaments, kontrahieren kann. Dies kann ein paar Stunden bis ein paar Tage nach der Entbindung der Fall sein. Bei der erfindungsgemäßen Vorrichtung weist die Haube eine Innenwand, eine Außenwand mit einer außen an der Außenwand angeordneten Zugvorrichtung und einen dazwischen gebildeten Innenraum auf. Bei der Zugvorrichtung kann es sich beispielsweise um einen Schlauch oder eine, insbesondere ungezwirnte, Kunststoffleine handeln. Dadurch kann die Vorrichtung nach einer Stoppung der Blutung besonders gut und ohne großen Kraftaufwand durch Ziehen an der Außenwand mittels der Zugvorrichtung vom Uterus abgelöst werden, weil sich die Außenwand im Verhältnis zur Innenwand verschieben kann. Um ein solches Verschieben der Außenwand im Verhältnis zur Innenwand zu erleichtern, kann im Innenraum ein Gleitmittel, wie z. B. Polydimethylsiloxan (= Dimeticon), enthalten sein. Beim Abziehen vom Uterus gelangt dabei die Innenwand an der Öffnung der Haube zunehmend auf die Außenseite. Die Zugvorrichtung kann durch eine dafür geschaffene oder beim Schließen des Bauchraums belassene kleine Öffnung in der Bauchdecke auf die Außenseite des Körpers geführt werden. Zum Abziehen der Vorrichtung von der Gebärmutter kann dann an der Zugvorrichtung gezogen und die ganze Vorrichtung durch die kleine Öffnung aus dem Körper herausgezogen werden. Die Öffnung kann dabei im Bereich der Grube des Bauchnabels geschaffen werden, so dass nach Schließen der Öffnung keine kosmetisch auffällige Narbe verbleibt. Diese Lokalisation der Öffnung hat den weiteren Vorteil, dass sie sich meist verhältnismäßig nahe am oberen Ende der Gebärmutter befindet. Ein operatives Öffnen des Bauchraums ist zum Entfernen der erfindungsgemäßen Vorrichtung nicht erforderlich. Die Maßnahme kann sowohl nach einer vaginalen Entbindung als auch nach einer Entbindung mittels Kaiserschnitt angewandt werden. Sie kann das Anlegen der oben erwähnten Ballontamponade, das Legen der Uteruskompressionsnähte und/oder die Notfall-Hysterektomie ersetzen. Die erfindungsgemäße Vorrichtung kann sehr schnell angewandt werden. Spezielle chirurgische Fachkenntnisse, wie sie z. B. zum Legen der Uteruskompressionsnähte oder der Hysterektomie erforderlich sind, sind dazu nicht erforderlich.

Die aus nur zwei Ballonen bestehende Vorrichtung gemäß der WO 2009/103298 A2 erfordert zur Kompression des Uterus stets einen geschlossenen Bauchraum, um für die Ballone einen Gegendruck bereitstellen zu können. Da im Bauchraum oft kaum Platz ist, die Ballone unterzubringen, richtig zu positionieren und zu füllen ist es schwierig, die Ballone richtig anzuwenden. Die Anwendung der Ballone erfordert verhältnismäßig viel Zeit. Darüber hinaus wird durch die Ballonform bedingt kein Gegendruck von oben auf den Uterus ausgeübt. Bei einer Kompression des Uterus durch Aufblasen der Ballone kann der Uterus nach oben hin ausweichen. Dies kann auch durch die transparente elastische Plastikfolie, welche die beiden Ballone miteinander verbindet, nicht verhindert werden, weil diese nicht die gesamte Gebärmutter abdeckt. Darüber hinaus besteht durch die Form der Ballone die Gefahr, dass diese nach oben hin abrutschen und die Gebärmutter dadurch nach unten drücken, ohne sie jedoch so zu komprimieren, dass eine Blutung dadurch gestoppt werden kann.

Die erfindungsgemäße Vorrichtung kann im Gegensatz dazu durch ihre Haubenform den gesamten oberen Bereich des Uterus abdecken und komprimieren, ohne dass es dazu eines Gegendrucks aus dem Bauchraum bedarf. Darüber hinaus kann die erfindungsgemäße Haube bei geöffnetem Bauchraum, etwa nach einem Kaiserschnitt, schnell über den Uterus gestülpt werden und dadurch eine Blutung sofort stoppen. Ein geschlossener Bauchraum zur Ausübung eines Gegendrucks ist dazu nicht erforderlich. Da uterine Blutungen nach einer Geburt sehr stark sein können, kann der Zeitgewinn durch die schnelle Anwendung lebensrettend sein.

Die Art der auf den Uterus ausgeübten Kompression ist bei der erfindungsgemäßen Vorrichtung vollkommen anders als bei der aus der WO 2009/103298 A2 bekannten Vorrichtung. Bedingt durch ihre Form komprimieren die beiden Ballone gemäß der WO 2009/103298 A2 den Uterus im mittleren Bereich am stärksten, so dass der Uterus dem Druck sowohl nach oben als auch nach unten nachgeben kann. Dies wird durch die erfindungsgemäße Vorrichtung verhindert, so dass dadurch ein zuverlässiges Stoppen einer uterinen Blutung erreicht werden kann. Durch die erfindungsgemäße Vorrichtung kann teilweise verhindert werden, dass ein Infektionsrisiko durch eine ansonsten erforderliche Gabe von Blutkonserven oder Gerinnungsfaktoren eingegangen werden muss. Auch das Risiko einer Immunreaktion oder eines anaphylaktischen Schocks nach Gabe von Blutkonserven oder Gerinnungsfaktoren kann dadurch vermieden werden. Wenn dennoch eine Bluttransfusion durchgeführt werden muss, ist das Risiko einer Infektion, einer Immunreaktion oder eines anaphylaktischen Schocks zumindest vermindert, weil nur eine geringere Menge an zu transfundierendem Blut als ohne die erfindungsgemäße Vorrichtung erforderlich ist.

Die Zugvorrichtung kann auf der geschlossenen Seite der Haube, insbesondere am Scheitelpunkt oder im Bereich eines Scheitelpunkts der Haube, angeordnet sein. Eine solche Anordnung ist besonders günstig, um die Vorrichtung, nachdem sie nicht mehr benötigt wird an der Zugvorrichtung, beispielsweise durch eine dafür nach dem Anbringen der Haube gelassene oder extra dafür geschaffene kleine Öffnung in der Bauchdecke herauszuziehen. Als Bereich des Scheitelpunkts der Haube wird der obere, d. h. geschlossene, Teil der nach unten offenen Haube verstanden. Der obere Teil kann dabei in einem zentral von der geschlossenen Seite zur Öffnung der Haube geführten Querschnitt höchstens das obere Viertel, insbesondere höchstens das obere Fünftel, insbesondere höchstens das oberes Sechstel, der Erstreckung vom Scheitelpunkt zur Öffnung der Haube umfassen.

Die Haube kann aus einem körperverträglichen Kunststoff bestehen. Auch die Zugvorrichtung kann aus einem körperverträglichen Kunststoff bestehen. Bei dem Kunststoff kann es sich um Latex handeln. Es gibt jedoch Frauen, die eine Latex-Unverträglichkeit haben. Eine Latex-Unverträglichkeitsreaktion kann vermieden werden, wenn als Kunststoff ein Silikon gewählt wird.

Bei einer Ausgestaltung der Vorrichtung ist die Haube so bemessen oder ausgestaltet, dass dadurch eine Kompression der Eierstöcke, zumindest weitgehend, vermieden oder gegenüber einer auf andere Bereiche des Uterus ausgeübten Kompression verringert werden kann. Dazu kann die Haube entweder verhältnismäßig kurz ausgestaltet sein, so dass sie nur über den oberen Teil der Gebärmutter ragt, die Eierstöcke jedoch freilässt. Alternativ kann auf gegenüberliegenden Seiten der Haube jeweils ein Bereich vorgesehen sein, in dem keine oder eine weniger starke Kompression der Gebärmutter erfolgt. Dieser Bereich kann beispielsweise als Ausbauchung oder Ausnehmung in der Haube ausgestaltet sein.

Die Haube kann sich zur Öffnung hin verjüngen. In einem zentral von der geschlossenen Seite zur Öffnung der Haube geführten Querschnitt durch die Haube kann eine lichte Weite von einer Seite zur gegenüberliegenden Seite der Haube in einem ersten Bereich, der näher an der Öffnung als an der geschlossenen Seite liegt, geringer sein als in dem zweiten Bereich, der näher an der geschlossenen Seite als an der Öffnung liegt. Da der Uterus in seinem oberen geschlossenen Bereich ein größeres Ausmaß aufweist als in seinem unteren Bereich, sitzt auch eine Haube, die sich zur Öffnung hin nicht verjüngt, an sich fest auf dem Uterus. Durch das Verjüngen zur Öffnung hin bzw. durch die geringere lichte Weite im ersten Bereich kann jedoch verhindert werden, dass die aufgesetzte Haube bei entsprechender mechanischer Belastung, insbesondere beim oder nach dem Füllen des Innenraums mit einem Fluid, ungewollt von der Gebärmutter abrutscht.

Die Haube kann achsensymmetrisch ausgebildet sein. Dies erleichtert eine schnelle Anwendung im Notfall, weil dadurch vor der Anwendung nicht erst eine bestimmte Orientierung der Haube ermittelt werden muss.

Um die erfindungsgemäße Vorrichtung nach der Anwendung gut vom Uterus lösen zu können, kann die Vorrichtung, insbesondere auf ihrer bei bestimmungsgemäßem Gebrauch zum Uterus weisenden Seite, eine antiadhäsive Beschichtung aufweisen. Die Beschichtung kann Polydimethylsiloxan (= Dimeticon), NatriumHyaluronat, Carboxymethylcellulose oder Heparin zum Verhindern eines Verklebens durch gerinnendes Blut umfassen.

Der Innenraum kann einen in der Außenwand angeordneten Zugang mit einem Schlauch aufweisen, so dass der Innenraum über den Zugang und den Schlauch mit einem Fluid gefüllt werden kann. Bei dem Fluid kann es sich um ein Gas, insbesondere Luft oder Kohlendioxid, oder eine Flüssigkeit, insbesondere eine physiologische Kochsalzlösung oder eine Pufferlösung, handeln. Am Ende des Schlauchs kann ein Ventil vorgesehen sein, das verschlossen werden kann, so dass ein durch das Fluid im Innenraum erzeugter Druck gehalten wird. Der Schlauch kann als die genannte Zugvorrichtung ausgebildet sein. Dazu können die Anbringung des Schlauchs an der Außenwand und der Schlauch selbst, z. B. durch eine ausreichende Wandstärke und durch die Wahl eines geeigneten Materials, so ausgebildet sein, dass sie der beim Abziehen der Haube von der Gebärmutter auftretenden Zugkraft standhalten. Durch das Füllen des Innenraums mit dem Fluid kann ein Druck aufgebaut werden, durch den der Uterus weiter komprimiert wird. Dadurch kann eine ansonsten nicht zu stoppende Blutung gegebenenfalls gestoppt werden. Darüber hinaus kann über das Fluid der auf den Uterus ausgeübte Druck reguliert werden. Der Schlauch kann auch nachdem die Vorrichtung über den Uterus gestülpt worden ist, gegebenenfalls nach Heraussaugen darin noch enthaltenen Fluids, verschlossen werden, um die Haube auf dem Uterus zu fixieren. Beim späteren Abziehen der Haube kann der Schlauch geöffnet werden, so dass das Abziehen durch Eindringen von Luft erleichtert wird. Durch das Eindringen der Luft wird das Verschieben der Außenwand im Verhältnis zur Innenwand erleichtert. Die Bildung eines sich beim Abziehen gegebenenfalls im Innenraum bildenden Vakuums wird dadurch vermieden.

Optional kann bei der Ausgestaltung der Vorrichtung mit dem, insbesondere am Scheitelpunkt oder im Bereich des Scheitelpunkts der Haube angeordneten, Schlauch im Innenraum, insbesondere an der Innenwand, insbesondere am Scheitelpunkt oder im Bereich des Scheitelpunkts der Innenwand, ein draht- oder seilförmiges Fixierungsmittel angeordnet und durch den Schlauch vom Innenraum über eine Dichtung im Schlauch nach außen geführt sein. Günstig ist es dabei auch, wenn der Schlauch verhältnismäßig fest oder sogar starr ist. Durch das Fixierungsmittel und dessen Fixierung kann beim Füllen des Innenraums mit dem Fluid erreicht werden, dass ein Abstand zwischen der Außenwand und Innenwand am Scheitelpunkt und im Bereich des Scheitelpunkts der Innenwand beim Befüllen des Innenraums mit dem Fluid nicht oder zumindest nicht wesentlich zunimmt. Dadurch kann ein je nach Ausgestaltung der Haube bestehendes Risiko vermindert werden, dass die Vorrichtung beim Befüllen mit Fluid durch den im Innenraum entstehenden Druck und die dadurch gegen den Uterus drückende Innenwand vom Uterus heruntergedrückt wird und dass die Vorrichtung im Bauchraum ein zu großes Volumen einnimmt. Zum Abziehen der Vorrichtung wird nach einem Ablassen des Fluids die Fixierung des Fixierungsmittels gelöst, so dass es beim Ziehen an dem Schlauch an der Dichtung in den Schlauch hineingezogen wird. Je nach Material und Ausgestaltung der Haube ist ein solches Fixierungsmittel jedoch nicht erforderlich.

Bei einer Ausgestaltung der erfindungsgemäßen Vorrichtung ist ein Mittel vorhanden, mittels dem über den Schlauch und den Zugang mittels des Fluids ein Überdruck im Innenraum erzeugt und/oder aufrecht erhalten werden kann. Im einfachsten Fall kann es sich bei dem Mittel um eine handelsübliche, großvolumige Spritze handeln. Es kann sich dabei aber auch um eine, insbesondere elektronisch gesteuerte, Pumpe, gegebenenfalls mit einem, insbesondere elektronisch gesteuerten, Druckentlastungsventil handeln. Zusätzlich kann ein transvaginal in den Uterus einzuführender Drucksensor vorhanden sein, der mit dem Mittel in Verbindung steht. Das Inverbindungstehen ist dabei so ausgestaltet, dass vom Drucksensor erfasste Daten an das Mittel, insbesondere eine elektronische Steuerung des Mittels, beispielsweise über ein Kabel oder über einen Sender, übermittelt werden. Der Überdruck kann dann mittels des Mittels in Abhängigkeit von einem vom Drucksensor erfassten Druck reguliert werden. Auf diese Weise ist es möglich, einen Druck zu erzeugen, der gerade ausreicht, um die Blutung zu stillen, so dass Eileiter und/oder Eierstöcke nicht unnötig belastet, d. h. komprimiert, werden.

Die Erfindung betrifft weiterhin ein Set, umfassend eine Mehrzahl von erfindungsgemäßen Vorrichtungen zur Verwendung zur Therapie einer uterinen Blutung bei einem Menschen, wobei die darin enthaltenen Hauben unterschiedliche Größen aufweisen. Dies hat den Vorteil, dass im Falle einer überraschend auftretenden postpartalen uterinen Blutung schnell eine optimal zu der jeweiligen Gebärmutter passende Haube ausgewählt werden kann. Die Größe der Gebärmutter kann individuell variieren. Insbesondere nach Mehrlingsgeburten ist die Gebärmutter deutlich größer als nach einer Geburt nur eines Kindes.

Nachfolgend wird die Erfindung anhand eines Ausführungsbeispiels und einer Zeichnung näher erläutert:

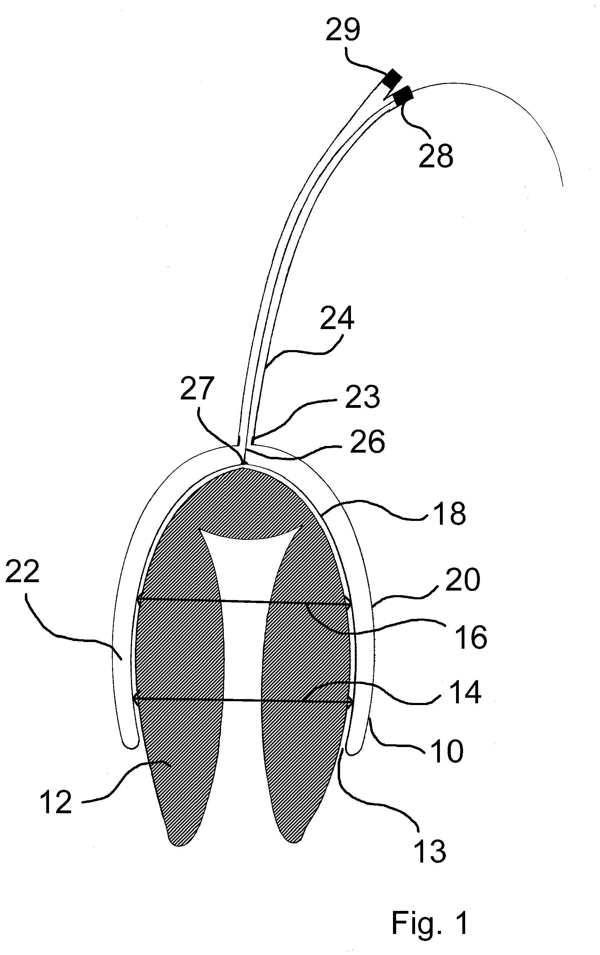

- Fig. 1: zeigt einen schematischen Querschnitt durch einen Uterus mit darübergestülpter erfindungsgemäßer Vorrichtung.

Fig. 1 zeigt eine erfindungsgemäße Haube 10, die über einen Uterus 12 gestülpt ist. Die Haube 10 weist eine Öffnung 13 auf. In dem hier dargestellten, zentral von der geschlossenen Seite zur Öffnung 13 der Haube 10 geführten Querschnitt durch die Haube 10 ist eine lichte Weite 14 von einer Seite zur gegenüberliegenden Seite der Haube 10 in einem ersten Bereich etwa gleich groß wie eine lichte Weite 16 in einem näher an der geschlossenen Seite als der Öffnung 13 liegenden zweiten Bereich. Die lichte Weite 14 im ersten Bereich kann jedoch auch geringer sein als die lichte Weite 16 im zweiten Bereich. Dadurch ist die Haube 10 dann unten enger, so dass sie nicht ungewollt vom Uterus abrutschen kann und einen besseren Halt hat.

Die Haube 10 weist eine Innenwand 18 und eine Außenwand 20 auf. Von der Innenwand 18 und von der Außenwand 20 wird der Innenraum 22 eingeschlossen, zu dem ein in der Außenwand 20 angeordneter Zugang 23 mit einem Schlauch 24 und einem Ventil 29 besteht. Über den Zugang 23, den Schlauch 24 und das Ventil 29 kann der Innenraum 22 mit einem Fluid gefüllt werden. Bei dem Fluid kann es sich um ein Gas oder um eine Flüssigkeit handeln. Nach dem Füllen mit dem Fluid kann zur Aufrechterhaltung des durch das Befüllen erzeugten Drucks das Ventil 29 geschlossen werden. Der Zugang 23 ist hier am Scheitelpunkt der Außenwand 20 angeordnet. Am Scheitelpunkt 27 der Innenwand 18 ist ein Fixierungsmittel 26 angeordnet. Dabei kann es sich beispielsweise um ein Kunststoffseil handeln. Das Fixierungsmittel 26 ist durch den Schlauch 24 über eine Dichtung 28 nach außen geführt. Das Fixierungsmittel 26 kann auf der Außenseite der Dichtung 28 fixiert werden. Es dient dazu, zu verhindern, dass sich der Abstand zwischen der Außenwand 20 und der Innenwand 18 der Haube 10 beim Befüllen des Innenraums 22 mit dem Fluid vergrößert und die Haube 10 durch den im Innenraum 22 entstehenden Druck und die dadurch gegen den Uterus 12 drückende Innenwand 18 vom Uterus 12 heruntergedrückt wird.

Zum späteren Abziehen der Haube 10 vom Uterus 12 wird die Fixierung das Fixierungsmittel 26 auf der Außenseite der Dichtung 28 gelöst, beispielsweise indem das Fixierungsmittel 26 durchgeschnitten wird oder eine zur Fixierung dienende Klemme gelöst wird. In den Innenraum 22 gefülltes Fluid kann durch Öffnen des Ventils 29 abgelassen werden. Anschließend wird die Haube 10 durch Ziehen am Schlauch 24 vom Uterus 12 abgezogen. Dabei rutscht das Fixierungsmittel 26 in den Schlauch 24. Durch das Eindringen von Luft über das Ventil 29 und den Schlauch 24 in den Innenraum 22 wird das Abziehen erleichtert. Die Haube 10 kann am als Zugvorrichtung dienenden Schlauch 24 durch eine kleine Öffnung in der Bauchdecke aus dem Bauchraum herausgezogen werden.

### Bezugszeichenliste

- 10: Haube
- 12: Uterus
- 13: Öffnung der Haube
- 14: lichte Weite in einem ersten Bereich
- 16: lichte Weite in einem zweiten Bereich
- 18: Innenwand
- 20: Außenwand
- 22: Innenraum
- 23: Zugang am Scheitelpunkt der Außenwand
- 24: Schlauch
- 26: Fixierungsmittel
- 27: Scheitelpunkt der Innenwand
- 28: Dichtung
- 29: Ventil

## Patentansprüche

1. Vorrichtung zur Verwendung zur Therapie einer uterinen Blutung bei einem Menschen, wobei die Vorrichtung eine Haube (10) aus einem sterilisierbaren Material umfasst, wobei die Haube (10) eine Form aufweist, die es erlaubt, die Haube (10) von außen so über einen Uterus (12) zu stülpen, dass dieser dadurch komprimiert wird, wobei die Haube (10) eine Innenwand (18), eine Außenwand (20) mit einer außen an der Außenwand (20) angeordneten Zugvorrichtung und einen zwischen der Innenwand (18) und der Außenwand (20) gebildeten Innenraum (22) aufweist, **dadurch gekennzeichnet, dass** das Material elastisch ist.

2. Vorrichtung nach Anspruch 1, wobei die Zugvorrichtung am Scheitelpunkt oder im Bereich eines Scheitelpunkts der Haube (10) angeordnet ist.

3. Vorrichtung nach einem der vorhergehenden Ansprüche, wobei das Material ein körperverträglicher Kunststoff ist.

4. Vorrichtung nach Anspruch 3, wobei der Kunststoff ein Silikon ist.

5. Vorrichtung nach einem der vorhergehenden Ansprüche, wobei die Haube (10) so bemessen oder ausgestaltet ist, dass dadurch eine Kompression der Eierstöcke, zumindest weitgehend, vermieden oder gegenüber einer auf andere Bereiche des Uterus (12) ausgeübten Kompression verringert werden kann.

6. Vorrichtung nach Anspruch 5, wobei die Haube (10) so kurz ausgestaltet ist, dass sie nur über den oberen Teil der Gebärmutter ragt und die Eierstöcke freilässt oder dass auf gegenüberliegenden Seiten der Haube (10) jeweils ein Bereich vorgesehen ist, in dem eine weniger starke oder keine Kompression der Gebärmutter erfolgt.

7. Vorrichtung nach einem der vorhergehenden Ansprüche, wobei sich die Haube (10) zur Öffnung (13) hin verjüngt.

8. Vorrichtung nach einem der vorhergehenden Ansprüche, wobei in einem zentral von der geschlossenen Seite zur Öffnung (13) der Haube (10) geführter Querschnitt durch die Haube (10) eine lichte Weite (14, 16) von einer Seite zur gegenüberliegenden Seite der Haube (10) in einem ersten Bereich, der näher an der Öffnung (13) als an der geschlossenen Seite liegt, geringer ist als in einem zweiten Bereich, der näher an der geschlossenen Seite als an der Öffnung (13) liegt.

9. Vorrichtung nach einem der vorhergehenden Ansprüche, wobei der Innenraum (22) einen in der Außenwand (20) angeordneten Zugang (23) mit einem Schlauch (24) aufweist, so dass der Innenraum (22) über den Zugang (23) und den Schlauch (24) mit einem Fluid gefüllt werden kann.

10. Vorrichtung nach Anspruch 9, wobei der Schlauch (24) als die Zugvorrichtung ausgebildet ist.

11. Vorrichtung nach Anspruch 9 oder 10, wobei im Innenraum (22), insbesondere an der Innenwand (18), insbesondere am Scheitelpunkt (27) oder im Bereich des Scheitelpunkts (27) der Innenwand (18), ein draht- oder seilförmiges Fixierungsmittel (26) angeordnet und durch den Schlauch (24) vom Innenraum (22) über eine Dichtung (28) im Schlauch (24) nach außen geführt ist.

12. Vorrichtung nach einem der Ansprüche 9 bis 11, wobei ein Mittel vorgesehen ist, mittels dem über den Schlauch (24) und den Zugang (23) mittels des Fluids ein Überdruck im Innenraum (22) erzeugt und/oder aufrechterhalten werden kann.

13. Vorrichtung nach Anspruch 12, wobei ein transvaginal in den Uterus (12) einzuführender Drucksensor vorgesehen ist, der mit dem Mittel in Verbindung steht, und der Überdruck mittels des Mittels in Abhängigkeit von einem vom Drucksensor erfassten Druck reguliert wird.

14. Set, umfassend eine Mehrzahl von Vorrichtungen nach einem der vorhergehenden Ansprüche zur Verwendung zur Therapie einer uterinen Blutung bei einem Menschen, wobei die in dem Set enthaltenen Hauben (10) unterschiedliche Größen aufweisen.

## Claims

1. A device for use for the therapy of uterine bleeding in a human, the device comprising a hood (10) made of a sterilizable material, the hood (10) having a shape that allows the hood (10) to be placed from the outside over a uterus (12) so as to compress the same, the hood (10) comprising an inner wall (18), an outer wall (20) having a pulling device associated with the outer wall (20) and an interior space (22) formed between the inner wall (18) and the outer wall (20), **characterized in that** the material is elastic.

2. The device according to claim 1, wherein the pulling device is arranged at the crest or in the region of a crest of the hood (10).

3. The device according to any one of the preceding claims, wherein the material is a body-compatible plastic material.

4. The device according to claim 3, wherein the plastic material is a silicone.

5. The device according to any one of the preceding claims, wherein the hood (10) is dimensioned or configured such that a compression of the ovaries thereby is, at least substantially, avoided or can be reduced compared to a compression exerted on other regions of the uterus (12).

6. The device according to claim 5, wherein the hood (10) is configured so short that it protrudes only over the upper portion of the womb and leaves the ovaries exposed, or that a respective region is provided on opposing sides of the hood (10) in which less strong or no compression of the womb takes place.

7. The device according to any one of the preceding claims, wherein the hood (10) tapers toward the opening (13).

8. The device according to any one of the preceding claims, wherein, in a cross-section through the hood (10) taken centrally from the closed side to the opening (13) of the hood (10), a clear width (14, 16) from one side to the opposing side of the hood (10) in a first region, which is located closer to the opening (13) than to the closed side, is less than in a second region, which is located closer to the closed side than to the opening (13).

9. The device according to any one of the preceding claims, wherein the interior space (22) comprises an access (23) that is arranged in the outer wall (20) and has a hose (24), so that the interior space (22) can be filled with a fluid via the access (23) and the hose (24).

10. The device according to claim 9, wherein the hose (24) is designed as a pulling device.

11. The device according to claim 9 or 10, wherein a wire- or cable-shaped fixation means (26) is arranged in the interior space (22), in particular on the inner wall (18), in particular at the crest (27) or in the region of the crest (27) of the inner wall (18), and is guided through the hose (24) from the interior space (22) via a seal (28) in the hose (24) to the outside.

12. The device according to any one of claims 9 to 11, wherein a means is provided by way of which overpressure can be generated and/or maintained in the interior space (22) via the hose (24) and the access (23) by way of the fluid.

13. The device according to claim 12, wherein a pressure sensor to be introduced transvaginally into the uterus (12) is provided and is connected to the means, and the overpressure is regulated by way of the means as a function of a pressure detected by the pressure sensor.

14. A set, comprising a plurality of devices according to any one of the preceding claims for use for the therapy of uterine bleeding in a human, wherein the hoods (10) contained in the set have differing sizes.

## Revendications

1. Dispositif en vue de l'utilisation pour la thérapie d'une hémorragie utérine chez l'homme, dans lequel le dispositif comprend une calotte (10) en un matériau stérilisable, dans lequel la calotte (10) présente une forme qui permet de retourner la calotte (10) de l'extérieur au-dessus d'un utérus (12) de sorte que celui-ci est comprimé ce faisant, dans lequel la calotte (10) présente une paroi interne (18), une paroi externe (20) avec un dispositif de traction disposé à l'extérieur sur la paroi externe (20) et un espace interne (22) formé entre la paroi interne (18) et la paroi externe (20), **caractérisé en ce que** le matériau est élastique.

2. Dispositif selon la revendication 1, dans lequel le dispositif de traction est disposé au sommet ou dans la région d'un sommet de la calotte (10).

3. Dispositif selon une des revendications précédentes, dans lequel le matériau est un plastique compatible avec le corps.

4. Dispositif selon la revendication 3, dans lequel le plastique est une silicone.

5. Dispositif selon une des revendications précédentes, dans lequel la calotte (10) est mesurée ou configurée de sorte qu'une compression des ovaires peut être ce faisant, au moins largement, évitée ou réduite par rapport à une compression exercée sur d'autres régions de l'utérus (12).

6. Dispositif selon la revendication 5, dans lequel la calotte (10) est configurée de manière aussi courte qu'elle ne dépasse qu'au-delà de la partie supérieure de l'utérus et libère les ovaires ou qu'une région dans laquelle une compression moins forte ou aucune compression de l'utérus (n')a lieu est à chaque fois prévue sur des côtés opposés de la calotte (10).

7. Dispositif selon une des revendications précédentes, dans lequel la calotte (10) rétrécit vers l'ouverture (13).

8. Dispositif selon une des revendications précédentes, dans lequel un écartement (14, 16) d'un côté au côté opposé de la calotte (10) est plus faible dans une première région qui se situe plus près de l'ouverture (13) que du côté fermé que dans une seconde région qui se situe plus près du côté fermé que de l'ouverture (13) dans une section transversale à travers la calotte (10) guidée centralement du côté fermé à l'ouverture (13) de la calotte (10).

9. Dispositif selon une des revendications précédentes, dans lequel l'espace interne (22) présente un accès (23) disposé dans la paroi externe (20) avec un tuyau (24) de sorte que l'espace interne (22) peut être rempli d'un fluide par le biais de l'accès (23) et du tuyau (24).

10. Dispositif selon la revendication 9, dans lequel le tuyau (24) est réalisé en tant que dispositif de traction.

11. Dispositif selon la revendication 9 ou 10, dans lequel un moyen de fixation en forme de fil ou câble (26) est disposé dans l'espace interne (22), notamment sur la paroi interne (18), notamment au sommet (27) ou dans la région du sommet (27) de la paroi interne (18) et est guidé à travers le tuyau (24) de l'espace interne (22) par le biais d'un joint (28) dans le tuyau (24) vers l'extérieur.

12. Dispositif selon une des revendications 9 à 11, dans lequel un moyen est prévu au moyen duquel une surpression peut être générée et/ou maintenue dans l'espace interne (22) par le biais du tuyau (24) et de l'accès (23) au moyen du fluide.

13. Dispositif selon la revendication 12, dans lequel un capteur de pression à introduire par voie transvaginale dans l'utérus (12) est prévu, lequel est connecté au moyen, et la surpression est régulée au moyen du moyen en fonction d'une pression détectée par le capteur de pression.

14. Ensemble comprenant une pluralité de dispositifs selon une des revendications précédentes en vue de l'utilisation pour la thérapie d'une hémorragie utérine chez l'homme, dans lequel les calottes (10) contenues dans l'ensemble présentent différentes tailles.
